**Europäisches Patentamt**

**European Patent Office**

**Office européen des brevets**

(19)

(11) Publication number: **0 160 282**

**B1**

(12)

# EUROPEAN PATENT SPECIFICATION

(45) Date of publication of patent specification: **17.01.90**

(51) Int. Cl.⁵: **G 01 N 21/07, B 04 B 5/04**

(21) Application number: **85105106.0**

(22) Date of filing: **26.04.85**

(54) Processor card for centrifuge.

(30) Priority: **03.05.84 US 606785**

(43) Date of publication of application:
**06.11.85 Bulletin 85/45**

(45) Publication of the grant of the patent:
**17.01.90 Bulletin 90/03**

(84) Designated Contracting States:
**AT BE CH DE FR GB IT LI LU NL SE**

(56) References cited:
**EP-A-0 039 825**
**EP-A-0 073 512**
**US-A-4 256 696**

(73) Proprietor: **ABBOTT LABORATORIES**
**14th Street and Sheridan Road North St**
**North Chicago Illinois 60064 (US)**

(72) Inventor: **Holen, James T.**
**938 Dublin Drive**
**Mundelein Illinois 60060 (US)**

(74) Representative: **Modiano, Guido et al**
**MODIANO, JOSIF, PISANTY & STAUB Modiano**
**& Associati Via Meravigli, 16**
**I-20123 Milano (IT)**

Courier Press, Leamington Spa, England.

## Description

This invention relates to a sample processor card for carrying out chemical analysis under centrifugal force.

In EP—A—0 160 901, filed on the same day as present application, there is described apparatus for carrying out chemical testing in which samples and/or reagents are manipulated by means of centrifugal force. The reagents and samples are placed in a sample processor device which is then placed in a centrifuge and subjected to high centrifugal forces. Manipulation of the reagents and samples in the sample processing device is achieved by rotating the device relative to the centrifuge itself so that the direction of centrifugal force acting on the device is changed.

The present invention relates to the sample processor device.

Known from EP—A—0,073,512 is an instrument for centrifugal analysis of fluids wherein inserts contain a plurality of chambers aligned generally radially with respect to the centrifuge. A sample is added at a radially inward position and, through alternating action of centrifugal force and gravity, moves radially outwardly and vertically. However, in order to achieve vertical movement relative to radial movement, it is necessary to slow the centrifuge to reduce the effect of centrifugal force. Conversely, to obtain radial movement relative to vertical movement of fluid, it is necessary to speed up the centrifuge. Consequently, the slowing and accelerating of the centrifuge causes undue wear on the drive mechanism and introduces anomalies in the flow dynamics of small volumes of fluid.

Also known from EP—A—0,039,825 is a centrifuge for fluid analysis having a platter which is rotated to move fluid throughout a plurality of chambers in radial and/or vertical directions. However, also in this prior device, centrifuge speed is alternately slowed and accelerated in order to balance centrifugal versus gravitational forces to achieve fluid movement, with attendant wear of the centrifuge drive mechanism and anomalies in the flow dynamics of the fluids.

Accordingly, it is an object of the present invention to overcome the problems encountered in the prior art by providing a sample processing card for use in a centrifuge of the type described in EP—A—0 160 901 for carrying out chemical testing.

It is a more specific object of the invention to provide a sample processing device in which chemical testing of a sample can be carried out under the effect of centrifugal force.

It is a further object of the invention to provide a sample processing device wherein the device can be provided with a stored reagent therein, ready for use in response to application of centrifugal force to the device, in which a chemical test can be carried out supplying a sample thereto and then applying centrifugal forces acting in two or more directions thereto to effect transfer of liquids from one chamber therein to another.

These and other objects and advantages of the invention are achieved by a sample processor card for carrying out analyses under centrifugal force, as defined in claim 1.

Further details and advantages of the invention will become apparent from the following detailed description of some embodiments thereof, given for purposes of illustration but not of limitation, together with the accompanying illustrative, non-limitative drawings, wherein:

Fig. 1 is a top view of a schematic diagram of centrifuge apparatus employed in the practice of the invention;

Fig. 2 is a side elevational view partially broken away of the apparatus shown in Fig. 1;

Fig. 3 is a plan view of a preferred form of the sample processor card of the invention;

Fig. 4 is a sectional view taken along the line 4—4 in Fig. 3;

Fig. 5 is a sectional view like that of Fig. 4, after the application of centrifugal force thereto;

Fig. 6 is an exploded view of the reagent container of the invention.

The concepts of the present invention reside in a sample processor card wherein the sample processor card is formed of a substantially closed chamber which includes a supply of reagent therein. The card includes inlet means for supplying a sample to the card, capillary means communicating with the inlet means to receive a sample supplied to the card and overflow means communicating with the capillary means to receive excess sample which is advanced from the inlet means through the capillary means under the influence of centrifugal force applied to the card in a first direction. The card also includes holding chamber means adapted to receive reagent from the reagent supply and sample from the capillary means in response to centrifugal force acting on the card in a second direction, and cuvette means communicating with the holding chamber means which is adapted to permit the measurement of the chemical reaction between the reagent and the sample. Thus, in the use of the sample processor card of the invention, flow of the reagent and the sample within the card is achieved solely by centrifugal force acting in two or more directions on the card as the card is subjected to high centrifugal forces in a centrifuge.

The sample processor card of the present invention can be used in any of a wide variety of analytical chemical techniques, including testing to determine blood chemistries, immunological testing for analyzing fluids and particularly body fluids as well as a number of other liquid analytical chemical techniques. The card of the present invention finds particular application in testing to determine blood chemistries in which the sample supplied to the card is a whole blood sample.

In accordance with that preferred embodiment of the invention, the card of the present invention also includes a sample separating chamber which communicates with the capillary means to separate the solid constituents of blood from the liquid constituents. Thus the sample separating chamber is positioned to receive sample from the capillary means which is caused to flow into the sample separating chamber under the influence of centrifugal force and therein caused to be separated by the centrifugal force. By providing the card with a sample separating chamber, it is possible to supply to the card a sample of whole blood which has not been previously spun down to separate liquid constituents from the solid constituents. That enables an operator to avoid a separate manipulative step of separating the whole blood before subjecting the sample of whole blood to chemical analysis.

Because most blood chemistry tests require the use of precisely measured samples, in the preferred practice of the invention, the sample processor card also includes a sample measuring chamber communicating with the capillary means or the sample separating chamber which is adapted to receive a measured quantity of sample in response to centrifugal force applied to the card. The sample measuring chamber is positioned adjacent to a sample overflow chamber which receives sample in excess of that filling the sample measuring chamber, the excess sample being retained in the sample overflow chamber as the direction of the centrifugal force is changed to cause the measured sample to be displaced from the sample measuring chamber to the sample holding chamber where it is mixed with the reagent in carrying out the chemical test under the effect of centrifugal force.

In another preferred embodiment of the invention, the sample processor card is provided with a supply of reagent in the form of means for dispensing reagent in response to centrifugal force applied to the card. By providing the card with a built-in supply of reagent, the card can be used by supplying a sample thereto and then subjecting the card to the effect of centrifugal force to release the reagent for admixing with the sample to carry out the chemical testing operation. In the most preferred embodiment, the means for dispensing the reagent includes a reagent chamber which is adapted to contain the reagent and strippable sealing means closing the reagent chamber whereby the sealing means is stripped from the reagent chamber in response to the application of centrifugal force to the card to release the reagent.

It is sometimes desirable that the reagent, by reason of its stability characteristics, be packaged separately from a reagent diluent. In that preferred embodiment of the invention, the application of centrifugal force to the card can serve to release both reagent and diluent either simultaneously or sequentially.

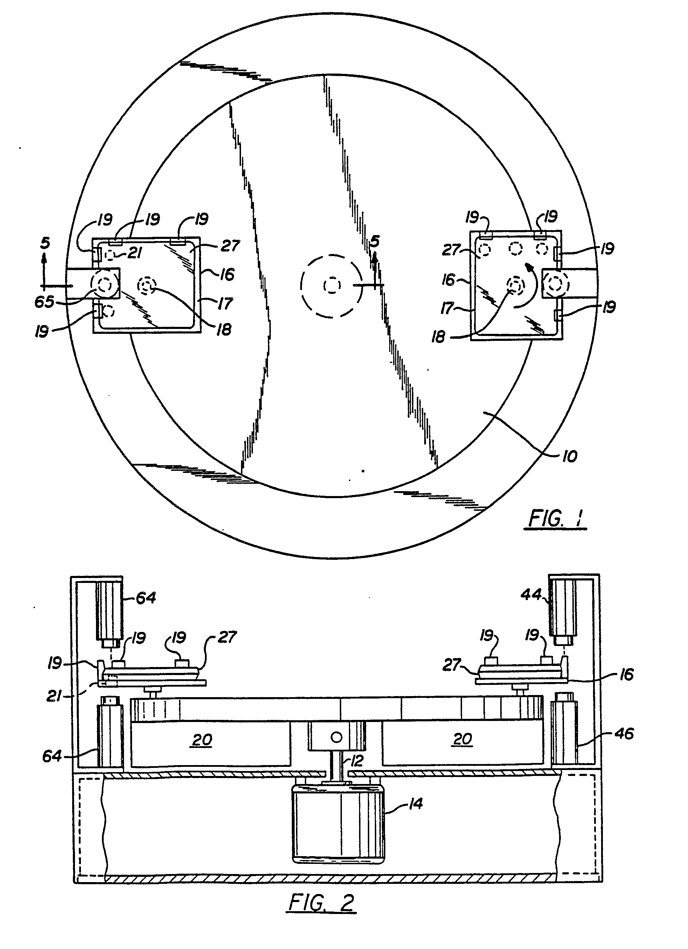

Referring now to the drawings for a more detailed description of the drawings, there is shown in Figs. 1—4 a schematic illustration of apparatus embodying the concepts of the present invention. The centrifuge includes a plate member 10 which is mounted on an axis 12 for rotation about the axis. The plate member 10 is preferably driven by suitable drive means 14 which may be, for example, an electric motor capable of operating at high speeds. While plate member 10 is shown in Fig. 1 as a circular plate, it will be understood that its configuration as shown is not critical to the practice of the invention. For example, it is equally possible to use a centrifugal arm mounted for rotation about an axis.

Mounted on plate member 10 is at least one sample processor card holder 16 adapted to receive a sample processor card described more fully hereinafter. As is shown in Figs. 1 and 2, the card holder 16 is in the nature of a tray and is rotatably mounted relative to the plate member 10 on an axis 18 operatively connected to means 20 to rotate the holder 16.

While the axis of rotation of the plate member 10 is illustrated in Fig. 2 as mounted on a vertical axis, it will be understood by those skilled in the art that the direction of the axis is not critical to the practice of the invention, and the axis, while preferably vertical, can also be horizontal or inclined in any direction since the effects of gravity on the sample processor card rotating with the plate member 10 is negligible.

In the preferred practice of the invention, the holder 16 can be rotated or indexed relative to the plate member 10 by any suitable drive means 20. In the preferred embodiment of the present invention, the holder 16 can be rotated or indexed 90° by the drive means 20. As will be appreciated by those skilled in the art, the holder 16 can be rotatable by an amount greater than 90° up to and including rotatable about a full 360°. The important feature is that the holder 16 adapted to receive the sample processor card be rotatable relative to the plate member 10 so that the direction of the centrifugal force acting on the sample processor card can be altered to effect the necessary fluid transport functions during the chemical testing operation.

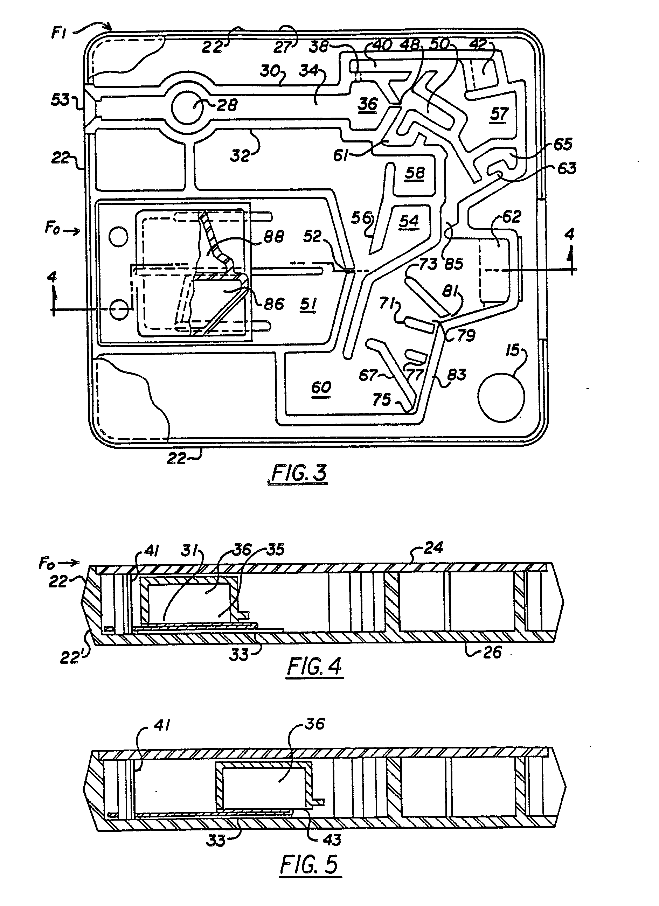

Referring to Figs. 3 to 5 for a description of the preferred sample processor card of the invention, there is shown a preferred sample processor card formed of a molded plastic article formed of outer walls 22 and 22' which, along with face plate 24 and bottom plate 26 define a unitary chamber. Within the chamber are a plurality of partitions defining the flow paths of the liquids during the chemical testing operation.

Sample can be introduced to the sample processor card by any of a variety of techniques. In accordance with one embodiment of the invention, the face plate 24 includes an opening 28 therein into which a blood sample, for example, may be deposited for analysis. Alternatively, there can be provided an opening 53 into which a capillary is placed to introduce a blood sample into a capillary slot 34 defined by two interior walls 30 and 32. In either case, blood introduced through the opening 28 or the opening 53 is moved through the capillary slot 34 by means of centrifugal force acting in the direction $F_0$ shown in Fig. 3.

As will be appreciated by those skilled in the art, the techniques involving the use of sample processor card 27 are applicable to any liquid to be subjected to chemical testing. In addition to whole blood, use can also be made of pre-spun blood fractions or other body fluids to be analyzed. Of course, the concepts of the present invention are equally applicable to other liquids which do not originate in the body on which chemical testing is conducted. For ease of description, however, the following describes the use of the sample card 27 using whole blood as the starting sample.

In the preferred practice of the invention, the sample processor card also includes a reagent chamber 86 and a diluent chamber 88 which operate, in response to centrifugal force acting in the direction $F_0$ as shown in Fig. 3, to release reagent and diluent. The essential feature of such a container is that it releases the diluent and reagent in response to centrifugal force acting upon the card 27.

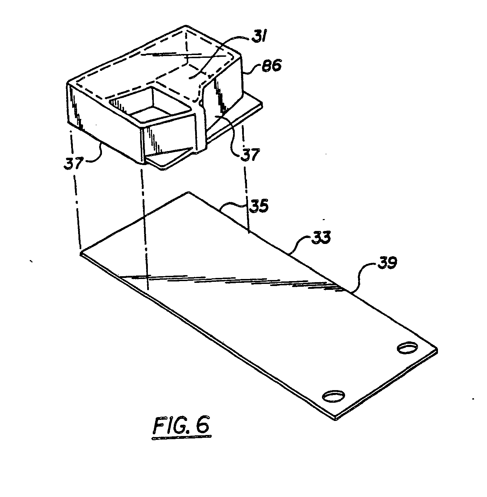

A preferred means for releasing the reagent is shown in Figs. 5 and 6. The reagent chamber 86 is a substantially closed container open at its lower portion 31. Closing that lower portion 31 is a removable strip 33 formed of a portion 35 adhered to the side walls 37 of chamber 86 and a portion 39 underlaying the portion 35 and fixed to the card 27 such as by means of a pin or pins 41.

As the card is subjected to centrifugal force in the direction $F_0$, the chamber 86 is displaced to the right as shown in Figs. 4 to 6, thereby peeling the removable strip 35 from the side walls 37 of the chamber 86 and releasing reagent through the opening 43 thus formed between the strip 35 and side walls 37.

In many chemical tests, it is preferred to package the reagent and a diluent therefor in separate chambers 86 and 88 as shown in Fig. 3. In the embodiment thus described, the strip 33 serves to seal the lower portions of both the reagent chamber 86 and the diluent chamber 88. The reagent and diluent chambers 86 and 88, being integral with each other, are displaced together in response to the application of centrifugal force to the card 27 and both reagent and diluent are thus released. Since the reagent chamber 86 is positioned slightly forward, in the direction $F_0$ of the centrifugal force, of the diluent chamber 88, the reagent is released prior to release of diluent.

Thus, in the use of the sample processor card of this invention, a blood sample is added to the card as described above, and then the card is positioned in the holder 16 in the centrifuge, insuring that the pin 21 for alignment of the sample processor card with the holder 16 passes through the corresponding key opening 15 extending through the sample card 27.

The card and the holder are positioned initially so that the blood well and reagent container are closest to the center of rotation of the plate member 10 to insure that the centrifugal force exerted on the sample processor card 27 during the initial rotation of plate member 10 is exerted in the direction $F_0$ as shown in Fig. 3 of the drawing. Thus, after the sample of blood is placed in the blood well and the plate member 10 rotated at high speed to develop centrifugal force, that centrifugal force serves to (a) release the diluent and reagent from their respective chamber 88 and 86, and, at the same, (b) move the blood sample inserted into the blood well 28 down the capillary slot 34 under the effect of the centrifugal force.

Downstream of the capillary slot 34 is a blood holding chamber 36 which is filled with the blood sample deposited into the card. Thus, the blood holding chamber 36 operates as a gross measure, selecting a predetermined quantity of blood sufficient to fill the chamber 50 as described hereinafter. Any blood in excess of the quantity filling chamber 36 passes through an opening 38 defined by a wall of the measuring chamber 36. Thus, the excess blood passes through an excess blood slot 40 to overflow chamber 42 located downstream of the excess blood slot 40. The presence of blood in the overflow chamber 42 can thus be used to confirm to the user that the blood sample deposited in the blood well was of a volume sufficient to completely fill the separating chamber 50.

In the preferred practice of the invention, it is frequently desirable to provide the apparatus with optical means positioned to detect the presence of blood in the overflow chamber 42 to thereby confirm that the sample provided was of a sufficient volume. For that purpose, the apparatus may include a source of light 44 and a detector 46, one or the other being positioned above the rotating plate 10 and the latter being positioned beneath the holder 16 in alignment with the opening 25 to detect the presence of blood in the overflow chamber 42.

In the preferred embodiment of the invention, the excess blood opening 38 is larger than the exit capillary 48 of the holding chamber 36 to insure that excess blood is rapidly discharged through the excess blood opening 38 and into the overflow chamber 42. Any quantity of blood in excess to the capacity of the overflow chamber 42 can thus spill over into an auxiliary blood overflow chamber 57.

As centrifugal force continues to act on the blood in the holding chamber 36, it is discharged into a blood separating chamber 50 in which blood is subjected to centrifugal force to separate the solid particulate matter from the fluid phase, any excess spilling over blood separating chamber 50 to the blood overflow chamber 42. As will be appreciated by those skilled in the art, the blood thus introduced to the separating chamber 50 is in effect spun down by the centrifugal force acting in the direction $F_0$ in Fig. 3 to separate the solid matter from the liquid, the solid matter being more dense than the liquid to thereby form a layer of solid matter at the lower portion of the blood separating chamber 50.

As will be appreciated by those skilled in the art, the release of the diluent and reagent from their respective chambers 88 and 86 can occur simultaneously with the movement by centrifugal force of the blood sample down the capillary slot 34. Alternatively, it is possible, and sometimes desirable, to provide a multi-speed operation, a lower speed below a threshold level at which the diluent and reagent are released but one at which the blood is still displaced downwardly through the capillary slot. That technique permits the blood to be separated in the blood separating 50 before the diluent and reagent are released from their respective chambers 88 and 86. Thus, after the blood has been separated in the blood separating chamber 50, the speed of the centrifuge can be increased to effect release of the diluent and reagent.

In either case, the particular configuration of the diluent and reagent chambers 88 and 86 permit the reagent to be released before the diluent. The reagent thus passes into the chamber 51, through the restricted opening 52 and into the reagent measuring chamber 54. The diluent, released after the initial release of the reagent, likewise passes into the chamber 51 and into the reagent measuring chamber 54, with any excess spilling over the baffle 56 into the reagent overflow chamber 58.

As will again be appreciated by those skilled in the art, alternatives with respect to the use of the reagent can be employed. For example, a solid reagent can be employed and positioned as a pellet in reagent measuring chamber 54 which is activated on release of the diluent as the diluent flows into the reagent measuring chamber 54. Other physical forms of reagent may likewise be used, such as a reagent gel, which would likewise be positioned in the reagent measuring chamber 54.

Alternatively, the solid reagent could be present as a coating on the walls of the reagent measuring chamber 54 which is dissolved when the diluent is released and passed into the reagent measuring chamber 54 as described above. Such a coating of reagent can also be applied to other areas of the card, notably the mixing chamber 60 and/or the cuvette chamber 62, both of which are described more fully hereinafter.

It is an important concept of the most preferred embodiment of the invention that the reagent measuring chamber 54 measures a precise, predetermined amount of reagent and diluent.

Once the reagent (mixed with diluent) has been measured in the reagent measuring chamber 54 and the blood separated in the blood separating chamber 50, the card is rotated 90° so that the centrifugal force is now acting in the direction $F_1$ as shown in Fig. 3. After rotation of the card, the centrifugal force thus displaces the measured quantity of reagent (mixed with diluent) from the reagent measuring chamber 54 to a mixing chamber 60. At the same time, the liquid constituent of the blood sample or a portion thereof is transferred to a sample holding chamber 61 downstream of the separating chamber 50. (Downstream as used in that sense is downstream in the direction of the centrifugal force when it is acting in the direction $F_1$ as shown in Fig. 3).

The sample card is then again rotated back to the original position where the centrifugal force is acting in the direction $F_0$ as shown in Fig. 3. In that position, the centrifugal force causes the sample in the sample holding chamber 61 to be conveyed to the sample measuring chamber 63, with any excess sample overflowing sample measuring chamber 63 to a sample overflow chamber 65.

Simultaneously, on rotation of the card to the position where the centrifugal force is acting in the direction $F_0$ as shown in Fig. 3, the reagent (mixed with diluent) in the mixing chamber 60 is displaced in a downstream direction. Positioned in the mixing chamber 60 are a series of baffles 67, 69, 71 and 73 which, along with the lateral wall 83 of the mixing chamber 60, define a series of restricted openings 75, 77, 79 and 81. The purpose of those restricted openings is to generate turbulence in the reagent (mixed with diluent) as it flows from the upper portion of the mixing chamber 60 toward the cuvette chamber 62, more fully described hereinafter. As the reagent (mixed with diluent) passes through those series of openings, the resulting turbulence insures that complete mixing of the diluent with the reagent will be achieved.

Thus the reagent is moved under the effect of the centrifugal force through the restricted openings 75, 77, 79 and 81 into the cuvette chamber 62. Since the reagent, at this stage of the operation, is unmixed with the sample, the sample remaining in the sample measuring chamber 63, the operator is permitted to take an optical reading of the reagent itself, prior to the time that it is mixed with the sample.

For the purpose of determining the optical characteristics of the reagent mixed with the diluent before contact with the sample, use can be made of a light source 64 and a light detector 64', one being positioned above the card holder 16 and the other beneath it, again with an opening in the card holder 16 to permit the transmission of light from the source 64 to the detector 64' through the cuvette chamber 62. That is sometimes a desirable operation, particularly when the measurements being taken on the sample are to be optical characteristics such as absorbance. The reading taken on the reagent before contact with the diluent enables one to correct the final readings for any absorbance contributed by the raw reagent. That technique can also be used to enable the operator to determine that the reagent was of high quality, and had not been degraded through the passage of time or by contact with an adverse environment.

After the operator has had an opportunity to monitor the characteristics of the reagent in the cuvette chamber 62, the sample processor card is again rotated 90° so that the centrifugal force is again acting in the direction $F_1$ as shown in Fig. 3 of the drawing. The centrifugal force thus causes the sample, in the sample measuring chamber 63, to pass through a chamber 85 and into the mixing chamber 60 where the sample, along with the reagent from the cuvette chamber 62, pass together through the series of restricted openings 81, 79, 77 and 75 into the upper portion of the mixing chamber 60 to effect mixing of the sample with the reagent. Because of the configuration of the baffle separating the sample measuring chamber 63 from the sample overflow chamber 65 any sample in the overflow chamber 65 is retained therein.

After the sample and reagent reach the upper portion of the mixing chamber 60, the card is again rotated 90° so that the centrifugal force is once again acting in the direction $F_0$. That rotation of the card causes the sample and reagent in the upper portion of the mixing chamber 60 to again pass through the restricted openings 75, 77, 79 and 81. In other words, mixing of the sample with the reagent occurs by means of two passes through the restricted openings 75 to 81 as described. The mixture of the sample and reagent is thus displaced under the centrifugal force acting in the direction $F_0$ into the cuvette chamber 62. At this stage in the procedure, optical readings of the reaction product of the sample and reagent can be taken incrementally or the final stage by means of the light source 64 and detector 64' in the manner described above.

Alternatively, continuous mixing can be achieved by again rotating the card so that the reagent and sample mixture is again displaced through the restricted openings while the chemical reaction between the two is ongoing during the incubation period of the reaction.

It is an important concept of the present invention that the centrifugal force operating on the fluids in the sample processor card be at a relatively high level so that the centrifugal force greatly overwhelms the fluid surface tension. That insures that the meniscus of the fluids defines a section of a substantially circular cylinder about the center of the centrifuge plate. When the sample processor card is rotated, the fluids pour from one chamber to another in the same way as if the chamber size and fluid quantities were much larger. If the rotation were such that substantially lower centrifugal forces were created, the fluids would tend to pour in large droplets and give quite variable results. It has accordingly been found that best results are usually achieved when the plate member is rotated at speeds sufficient to create centrifugal forces of at least 500 g's.

It will be understood that various changes and modifications can be made in the details of construction without departing from the scope of the invention as defined in the following claims.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. A sample processor card (27) for carrying out analyses under centrifugal force, the card (27) being substantially closed and having therein a supply of reagent, inlet means (28, 53) for supplying a sample to the card (27) capillary means (34) communicating with the inlet means (28, 53) to receive sample, overflow means (40, 42) communicating with the capillary means (34) to receive excess sample under the effect of centrifugal force applied to the card (27) in a first direction $(F_0)$, holding chamber means (60) for receiving the sample and reagent and cuvette means (62) communicating with the holding chamber means (60) for permitting measurement of the reaction between the sample and reagent, characterized in that the holding chamber means (60) is adapted to receive sample and reagent in response to changing the direction of centrifugal force applied relative to the card (27).

2. A card as defined in claim 1 characterized in that it comprises sample separating chamber means (50) communicating with the capillary means (34) and adapted to separate, under centrifugal force, constituents of the sample.

3. A card as defined in claim 1 or 2 characterized in that it comprises sample measuring chamber means (54) communicating with the capillary means (34) and adapted to receive a measured quantity of sample from the capillary means (34) in response to the application of centrifugal force to the card (27).

4. A card as defined in claim 1, 2 or 3 characterized in that said sample overflow means (40, 42) are positioned to receive sample in excess of that filling the sample measuring chamber means (63) and adapted to retain such excess sample.

5. A card according to one or more of the preceding claims, characterized in that the supply of reagent in the card includes means for dispensing reagent (86) in response to centrifugal force applied to the card.

6. A card according to claim 5 characterized in that the means for dispensing reagent (86) includes means for dispensing reagent (86) and means for dispensing diluent (88) for the reagent.

7. A card according to claim 5 or 6 characterized in that the means for dispensing reagent includes reagent chamber means (86) adapted to contain the reagent and strippable sealing means (33) closing the reagent chamber means (86) whereby the sealing means (33) is stripped from the reagent chamber means (86) in response to the application of centrifugal force applied to the card (27) to release reagent from the chamber means (86).

8. A card as defined in claim 7 characterized in that it comprises diluent chamber means (88) positioned adjacent to the reagent chamber means (86), the sealing means (33) closing the reagent chamber means (86) and the diluent chamber means (88) whereby the application of centrifugal force releases reagent and diluent.

9. A card as defined in claim 8 characterized in that the reagent chamber means (86) is positioned forward of the diluent chamber means (88) whereby the reagent is released prior to the release of diluent in response to the application of centrifugal force.

10. A card according to claim 7 or 8, characterized in that it includes means (41) for securing to the card (27) the sealing means (33) whereby the application of centrifugal force to the card (27) displaces the reagent chamber means (86) in the direction ($F_0$) of the centrifugal force to strip the sealing means (33) from the reagent chamber means (86) to thereby release the reagent.

11. A card according to one or more of the preceding claims, characterized in that movement of the sample and reagent throughout the processor card (27) is accomplished solely by changing the orientation of the processor card (27) relative to the centrifuge, without changing the speed or direction of rotation of the centrifuge.

**Patentansprüche**

1. Probenbehandlungskarte (27) zum Durchführen von Analysen unter Fliehkraft, wobei die Karte (27) im wesentlichen geschlossen ist und einen Vorrat eines Reagens enthält, mit Einlaßmitteln (28, 53), um der Karte (27) eine Probe zuzuführen, Kapillarmitteln (34), die mit den Einlaßmitteln (28, 53) zum Aufnehmen der Probe in Verbindung stehen, Überströmmitteln (40, 42), die mit den Kapillarmitteln (34) zum Aufnehmen eines Probenüberschusses in Verbindung stehen, der unter der Wirkung der auf die Karte (27) in einer ersten Richtung ($F_0$) ausgeübten Fliehkraft auftritt, Kammermitteln (60) zum Halten der Probe und des Reagens, und Küvettenmitteln (62), die mit den Kammerhaltemitteln (60) in Verbindung stehen, um eine Messung der Reaktion zwischen der Probe und dem Reagens zu gestatten, dadurch gekennzeichnet, daß die Kammerhaltemittel (60) so ausgebildet sind, daß sie die Probe und das Reagens bei einer Richtungsänderung der auf die Karte (27) ausgeübten Fliehkraft aufnehmen.

2. Karte nach Anspruch 1, dadurch gekennzeichnet, daß sie Kammermittel (50) zur Probentrennung aufweist, die mit den Kapillarmitteln (34) in Verbindung stehen und so ausgebildet sind, daß sie Bestandteile der Probe unter Fliehkraft voneinander trennen.

3. Karte nach Anspruch 1 oder 2, dadurch gekennzeichnet, daß sie Kammermittel (54) zur Probenmessung aufweist, die mit den Kapillarmitteln (34) in Verbindung stehen und so ausgebildet sind, daß sie bei Ausüben der Fliehkraft auf die Karte (27) eine abgemessene Probenmenge von den Kapillarmitteln (34) aufnehmen.

4. Karte nach einem der Ansprüche 1 bis 3, dadurch gekennzeichnet, daß die Probeüberströmmittel (40, 42) so angeordnet sind, daß sie beim Füllen der zur Probenmessung dienenden Kammermittel (63) anfallenden Überschuß aufnehmen, und so ausgebildet sind, daß sie einen derartigen Probenüberschuß zurückhalten.

5. Karte nach einem der vorhergehenden Ansprüche, dadurch gekennzeichnet, daß der Reagentienvorrat in der Karte Mittel (86) zum Abgeben des Reagens auf Grund der auf die Karte ausgeübten Fliehkraft aufweist.

6. Karte nach Anspruch 5, dadurch gekennzeichnet, daß die Mittel (86) zum Abgeben des Reagens sowohl Reagensabgabemittel (86) als auch Mittel (88) zum Abgeben eines Verdünners für das Reagens umfassen.

7. Karte nach Anspruch 5 oder 6, dadurch gekennzeichnet, daß die Reagensabgabemittel Reagens-Kammermittel (86), die zur Aufnahme des Reagens ausgebildet sind und abstreifbare Dichtungsmittel (33) aufweisen, welche die Reagens-Kammermittel (86) verschließen, wobei die Dichtungsmittel (33) von den Reagens-Kammermitteln (86) bei Ausüben einer Fliehkraft auf die Karte (27) zum Freisetzen des Reagens aus den Kammermitteln (86) abgestreift werden.

8. Karte nach Anspruch 7, dadurch gekennzeichnet, daß sie Verdünner-Kammermittel (88) aufweist, die den Reagens-Kammermitteln (86) benachbart sind, wobei Dichtungsmittel (33) die Reagens-Kammermittel (86) und die Verdünner-Kammermittel (88) verschließen und wobei bei Ausüben einer Fliehkraft das Reagens und der Verdünner freigesetzt werden.

9. Karte nach Anspruch 8, dadurch gekennzeichnet, daß die Reagens-Kammermittel (86) vor den Verdünner-Kammermitteln (88) angeordnet sind, so daß das Reagens bei Ausüben der Fliehkraft vor dem Verdünner freigesetzt wird.

10. Karte nach Anspruch 7 oder 8, dadurch gekennzeichnet, daß sie Mittel (41) zum Befestigen der Dichtungsmittel (33) an der Karte (27) aufweist, wobei die Reagens-Kammermittel (86) bei Ausüben der Fliehkraft auf die Karte (27) in der Fliehkraftrichtung ($F_0$) verlagert werden, um die Dichtungsmittel (33) von den Reagens-Kammermitteln (86) abzustreifen und damit das Reagens freizusetzen.

11. Karte nach einem der vorhergehenden Ansprüche, dadurch gekennzeichnet, daß die Bewegung der Probe und des Reagens durch die Behandlungskarte (27) hindurch lediglich durch Änderung der Orientierung der Behandlungskarte (27) relativ zur Zentrifuge erfolgt, ohne Änderung der Geschwindigkeit oder Drehrichtung der Zentrifuge.

**Revendications**

1. Carte (27) de traitement d'échantillon destinée à l'exécution d'analyses sous l'action d'une force centrifuge, la carte (27) étant pratiquement fermée et ayant une réserve d'un réactif, un dispositif d'entrée (28, 53) destiné à transmettre un échantillon à la carte (27), un dispositif capillaire (34) communiquant avec le dispositif d'entrée (28, 53) afin qu'il reçoive l'échantillon, un dispositif de débordement (40, 42) communiquant avec le dispositif capillaire (34) et destiné à recevoir un excès d'échantillon sous l'action de la force centrifuge appliquée à la carte (27) dans une première direction ($F_0$), une chambre (60) de retenue destinée à recevoir l'échantillon et le réactif, et un dispositif à cellule (62) communiquant avec la chambre de retenue (60) et destiné à permettre la mesure de la réaction entre l'échantillon et le réactif, caractérisée en ce que la chambre de retenue (60) est destinée à recevoir l'échantillon et le réactif à la suite du changement de direction de la force centrifuge appliquée à la carte (27).

2. Carte selon la revendication 1, caractérisée en ce qu'elle comporte une chambre (50) de séparation d'échantillon communiquant avec le dispositif capillaire (34) et destinée à séparer des constituants de l'échantillon sous l'action de la force centrifuge.

3. Carte selon la revendication 1 ou 2, caractérisée en ce qu'elle comporte une chambre (54) de mesure d'échantillon communiquant avec le dispositif capillaire (34) et destinée à recevoir une quantité mesurée de l'échantillon provenant du dispositif capillaire (34) à la suite de l'application d'une force centrifuge à la carte (27).

4. Carte selon la revendication 1, 2 ou 3, caractérisée en ce que le dispositif (40, 42) de débordement de l'échantillon est disposé afin qu'il reçoive l'excès d'échantillon qui a rempli la chambre (63) de mesure d'échantillon et qu'il retienne cet excès d'échantillon.

5. Carte selon une ou plusieurs des revendications précédentes, caractérisée en ce que la réserve de réactif placée dans la carte comporte un dispositif de distribution du réactif (86) sous l'action de la force centrifuge appliquée à la carte.

6. Carte selon la revendication 5, caractérisée en ce que le dispositif de distribution du réactif (86) comporte un dispositif destiné à distribuer le réactif (86) et un dispositif destiné à distribuer un diluant (88) du réactif.

7. Carte selon la revendication 5 ou 6, caractérisée en ce que le dispositif de distribution du réactif comporte une chambre (86) de réactif destinée à contenir le réactif et un dispositif séparable (33) de fermeture étanche qui ferme la chambre de réactif (86) afin que le dispositif de fermeture étanche (33) se sépare de la chambre de réactif (86) sous l'action de la force centrifuge appliquée à la carte (27) afin que le réactif soit libéré de la chambre (86).

8. Carte selon la revendication 7, caractérisée en ce qu'elle comporte une chambre (88) de diluant, adjacente à la chambre de réactif (86), le dispositif de fermeture étanche (33) fermant la chambre de réactif (88) et la chambre de diluant (88) si bien que l'application de la force centrifuge provoque la libération du réactif et du diluant.

9. Carte selon la revendication 8, caractérisée en ce que la chambre de réactif (86) est disposée en avant de la chambre de diluant (88) afin que le réactif soit libéré avant la libération du diluant lors de l'application de la force centrifuge.

10. Carte selon la revendication 7 ou 8, caractérisée en ce qu'elle comporte un dispositif (41) de fixation du dispositif d'étanchéité (33) à la carte (27) afin que l'application d'une force centrifuge à la carte (27) provoque un déplacement de la chambre de réactif (86) dans la direction $(F_0)$ de la force centrifuge afin que le dispositif d'étanchéité (33) soit séparé de la chambre de réactif (86) et libère le réactif.

11. Carte selon une ou plusieurs des revendications précédentes, caractérisée en ce que le déplacement de l'échantillon et du réactif dans la carte de traitement (27) est réalisé uniquement par changement de l'orientation de la carte de traitement (27) par rapport à la centrifugeuse, sans changement de la vitesse ou du sens de rotation de la centrifugeuse.

FIG. 1

FIG. 2

FIG. 3

FIG. 4

FIG. 5

_FIG. 6_